# EUROPEAN PATENT APPLICATION

(11) **EP 3 896 413 A1**
(43) Date of publication of application: **20.10.2021**
(21) Application number: 19897022.0
(22) Date of filing: 12.12.2019
(51) Int. Cl.: G01J 5/00, A61B 5/01

(54) **EAR THERMOMETER**

(30) Priority: 14.12.2018 JP 2018234340
(71) Applicant: Bio Echo Net Inc., Sapporo-shi, Hokkaido 064-0804 (JP)
(72) Inventor: TANAKA, Hideki, Sapporo-shi, Hokkaido 064-0804 (JP)
(74) Representative: Granleese, Rhian Jane
(86) International application number: PCT/JP2019/048715
(87) International publication number: WO 2020/122181

(57) **Abstract**

An ear thermometer (E1 - E5) includes a probe (PB) including an infrared sensor (SM1) for measuring a temperature of an eardrum (250) of a target person in a non-contact manner and attached to an ear hole of the target person. The probe (PB) includes a probe body (20) inserted into the ear hole, a housing (10) supporting the probe body (20), and an in-ear type earpiece (12) attached to the probe body (20) and coming into contact inside the ear hole. The earpiece (12) includes a base portion (12a) including an engaging portion (12c) engaging with the probe body (20) and partially exhibiting a hollow conical shape, and a substantially cylindrical tip portion (12b) provided at one end of the base portion (12a) and extending in a direction away from the housing (10). The base portion (12a) and the tip portion (12b) are integrally formed of a flexible material. An outer diameter (L1) of the tip portion (12b) is set to be smaller than a maximum outer diameter (L2) of the base portion.

## Description

### TECHNICAL FIELD

The present invention relates to a thermometer that measures a body temperature of a temperature measurement target person, and more particularly to an ear thermometer that measures a temperature of an eardrum by inserting a temperature measuring unit into an ear hole.

### BACKGROUND

For example, in an operating room, an intensive care unit or the like, it is essential to measure a body temperature of a temperature measurement target person during the operation.

Further, for example, it may be necessary to measure a body temperature of a worker who performs heavy physical work for a long time as a part of physical condition management.

Since it is necessary to continuously measure a body temperature of a temperature measurement target person such as a patient and a worker, for a long time, it is important that a physical burden is small.

As a thermometer that meets such demands, an ear thermometer that measures a temperature of an eardrum by inserting a probe into an ear hole of a temperature measurement target person has been proposed (see PTL 1).

### CITATION LIST

### Patent Literature

PTL 1: JP 5039618 B2

### SUMMARY

Here, an ear thermometer has a shape similar to a shape of an earphone called a canal type. This is because both the ear thermometer and the canal type earphone are devices that are placed in an ear canal for use.

That is, the ear thermometer and the canal type earphone are similar in that each of those has a body portion (housing) to be worn between an auricle and a tragus, which are the outside of the ear canal, and an earpiece inserted into the ear canal.

However, the functions and purposes required of earpieces are different between the ear thermometer and the canal type earphone.

An earpiece of the canal type earphone has a shape for the purpose of efficiently transmitting a sound emitted from a main body to an eardrum through an ear canal.

That is, the earpiece of the canal type earphone has a shape that can efficiently transmit a small sound to an eardrum by sealing the inside of the ear canal so as to be in a constant pressure state and creating a tunnel effect.

On the other hand, the earpiece of the ear thermometer has an important function of directing infrared rays emitted from an eardrum so that an infrared sensor can receive the infrared rays.

In addition, the earpiece of the ear thermometer is required to have a function of stably holding the direction in which an infrared sensor can receive the infrared rays emitted from an eardrum during wearing.

Due to these differences in functions and purposes, there is a problem that infrared rays emitted from an eardrum cannot be detected efficiently even if the earpieces of the canal type earphones variously provided are merely applied to an earpiece of an ear thermometer.

In addition, an ear thermometer that satisfies the function of stably holding infrared rays, which are emitted from an eardrum, in the direction in which an infrared sensor can efficiently receive the infrared rays has not yet been developed.

The present invention has been made in view of the above problems, and an object of the present invention is to provide an ear thermometer capable of stably holding infrared rays, which are emitted from an eardrum, in a state in which an infrared sensor can efficiently receive the infrared rays.

In order to achieve the above object, an ear thermometer according to an aspect of the present invention includes a probe including an infrared sensor for measuring a temperature of an eardrum of a temperature measurement target person in a non-contact manner and attached to an ear hole of the temperature measurement target person. The probe includes a probe body inserted into the ear hole of the temperature measurement target person, a housing supporting the probe body, and an in-ear type earpiece attached to the probe body and coming into contact inside the ear hole of the temperature measurement target person. The earpiece includes a base portion including an engaging portion engaging with the probe body and partially exhibiting a hollow conical shape, and a substantially cylindrical tip portion provided at one end of the base portion and extending in a direction away from the housing. The base portion and the tip portion are integrally formed of a flexible material. An outer diameter of the tip portion is set to be smaller than a maximum outer diameter of the base portion.

This makes it possible to provide the ear thermometer capable of stably holding in the direction in which the infrared sensor can efficiently receive infrared rays emitted from an eardrum.

A total length of the earpiece in an insertion/removal direction into the ear hole may be set to have a length that reaches a second curve located on an inner side of an S-shaped curve of an ear canal.

As a result, the ear thermometer is capable of more stably holding in the direction in which the infrared sensor can efficiently receive the infrared rays emitted from the eardrum.

The infrared sensor may be arranged in a recess formed on a tip side of the probe body.

As a result, more accurate body temperature measurement can be performed by the infrared sensor arranged at a position close to the eardrum.

The probe body may include a cylindrical light guide tube in which a reflective material is arranged on an inner wall, and the infrared sensor may be arranged on a rear end side of the light guide tube.

As a result, the influence of noise on the infrared rays from the eardrum can be suppressed, therefore, more accurate body temperature measurement can be performed.

The housing may include an external sound intake hole for taking in an external sound on the probe body.

This makes it possible to transmit an external instruction or the like to the temperature measurement target person by sound.

The housing may include a sound output hole for emitting a sound taken in from the external sound intake hole to the earpiece, and the earpiece may be provided with a sound emitting portion for emitting a sound emitted from the sound output hole into the ear hole.

This makes it possible to more reliably transmit a sound such as an external instruction to the temperature measurement target person.

The ear thermometer may further include a sound output unit provided in the housing and outputting a sound by being connected to an external device.

As a result, an instruction or an alarm can be transmitted more clearly to the temperature measurement target person by sound via the external device.

The tip portion of the earpiece may be closed by a protective film.

This makes it possible to suppress the influence of thermal noise due to the fluctuation of air in the earpiece, thereby performing more accurate body temperature measurement.

With the ear thermometer according to the aspect of the present invention, the infrared rays emitted from the eardrum can be stably held in a state where the infrared sensor can efficiently receive the infrared rays.

### BRIEF DESCRIPTION OF THE DRAWINGS

FIG 1(a) is a right side view illustrating a configuration example of an ear thermometer according to a first embodiment, FIG 1(b) is a bottom view of the same, and FIG. 1(c) is a front view of the same.
FIG. 2 is a partial cross-sectional view illustrating a configuration example of the ear thermometer according to the first embodiment.
FIG. 3 is an explanatory view illustrating a dimension of the ear thermometer according to the first embodiment.
FIG. 4 is an explanatory view illustrating a schematic structure of a right ear to which the ear thermometer according to the first embodiment is attached.
FIG. 5 is a partial cross-sectional view illustrating another configuration example of the ear thermometer according to the first embodiment.
FIG. 6 is an explanatory view illustrating a state in which the ear thermometer according to the first embodiment is attached to a right ear.
FIG. 7 is an explanatory view illustrating a state in which a canal type earphone as a comparison target is attached to a right ear.
FIG. 8 is an explanatory view illustrating a state in which an ear thermometer according to a second embodiment is attached to a right ear.
FIG. 9 is a perspective view illustrating a configuration example of an earpiece applicable to the ear thermometer according to a third embodiment.
FIG. 10(a) is a top view illustrating a configuration example of an ear thermometer according to a fourth embodiment, FIG. 10(b) is a right side view of the same, and FIG. 10(c) is a bottom view of the same.
FIG. 11 is a right side view illustrating a state in which an earpiece is attached to the ear thermometer according to the fourth embodiment.
FIG. 12 is a cross-sectional view illustrating a configuration example of the ear thermometer according to the fourth embodiment.

### DETAILED DESCRIPTION

### (First embodiment)

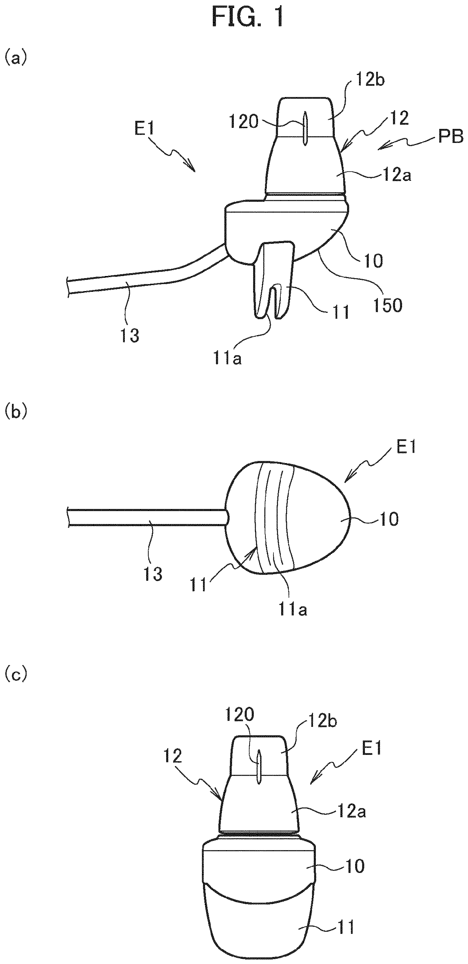

With reference to FIGS. 1 to 7, an ear thermometer E1 (E2) according to a first embodiment will be described.

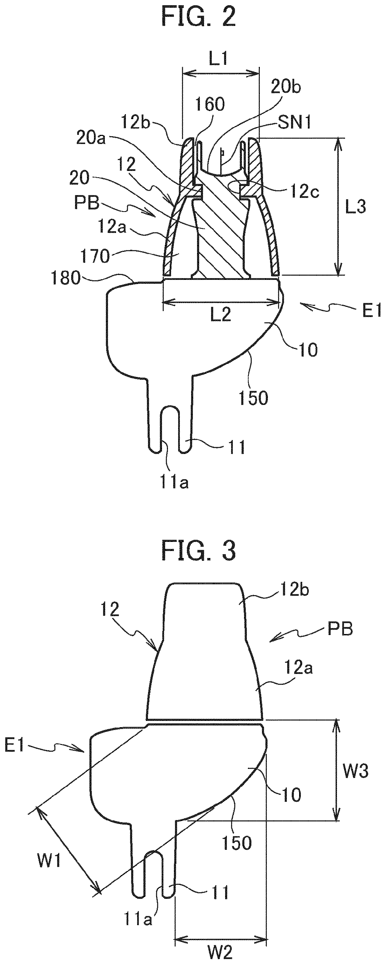

FIG. 1(a) is a right side view illustrating a configuration example of the ear thermometer E1 according to a first embodiment, FIG. 1(b) is a bottom view illustrating a configuration example of the ear thermometer E1 according to the first embodiment, and FIG. 1(c) is a front view illustrating a configuration example of the ear thermometer E1 according to the first embodiment. FIG. 2 is a partial cross-sectional view illustrating a configuration example of the ear thermometer E1 according to the first embodiment. FIG. 3 is an explanatory view illustrating a dimension of the ear thermometer E1 according to the first embodiment.

As illustrated in FIGS. 1 to 3, the ear-type thermometer E1 according to the first embodiment includes an infrared sensor SN1 for measuring a temperature of an eardrum of a temperature measurement target person in a non-contact manner, and a probe PB attached to an ear hole of a temperature measurement target person.

The probe PB mainly includes a probe body 20 inserted into an ear hole of a temperature measurement target person, a housing 10 that supports the probe body 20, and an in-ear type earpiece 12 that is attached to the probe body 20 and comes into contact inside the ear hole of the temperature measurement target person.

The housing 10 and the probe body 20 are molded of ABS resin or the like. The housing 10 and the probe body 20 may be integrally molded.

In the ear thermometer E1 according to the first embodiment, as illustrated in FIG. 2, the infrared sensor SN1 is arranged in a recess 20b formed on the tip side of the probe body 20.

As a result, more accurate body temperature measurement can be performed by the infrared sensor SN1 arranged at a position close to an eardrum 250.

As illustrated in FIG. 2 and the like, the earpiece 12 includes an engaging portion (protrusion portion) 12c that engages with a groove portion 20a on the probe body 20. Further, the earpiece 12 includes a base portion 12a that partially exhibits a hollow conical shape, and a substantially cylindrical tip portion 12b extending in a direction away from the housing 10 while provided at one end of the base portion 12a.

The base portion 12a and the tip portion 12b are integrally formed of a soft and flexible material such as silicone rubber.

An outer diameter L1 of the tip portion 12b is set to be smaller than a maximum outer diameter L2 of the base portion 12a.

Due to the shape of the earpiece 12 and the shape of the housing 10 as described above, the ear thermometer E1 according to the first embodiment is stably held in a state where it can efficiently receive infrared rays from the eardrum. Specific examples of dimensions and holding states will be described later.

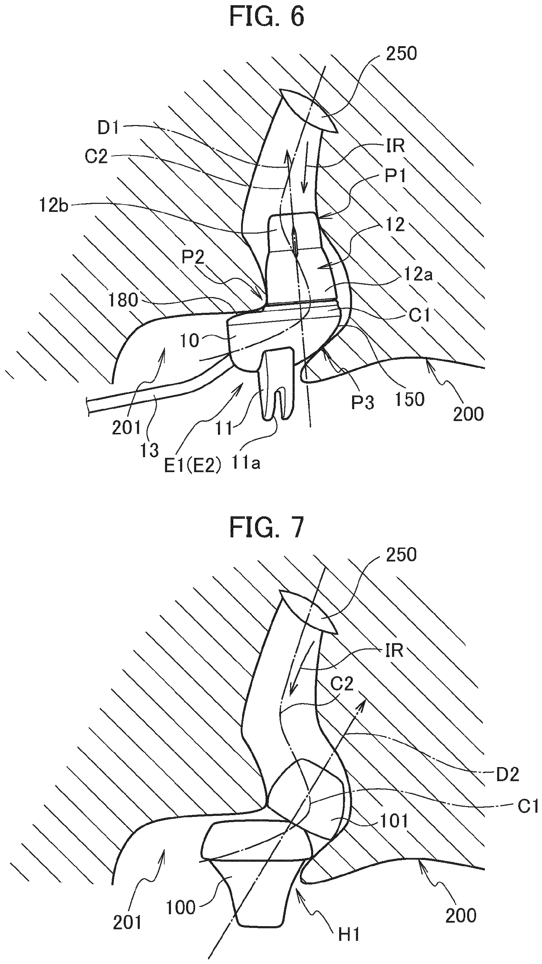

As illustrated in FIG. 6, a total length L3 of the earpiece 12 in the insertion/removal direction into the ear hole is set to have a length that reaches a curve (second curve) C2 located on the inner side of an S-shaped curve of an ear canal 201.

### (Regarding Dimensions of L1, L2 and L3)

The dimensions of L1 to L3 are set as follows:
a) Take a mold in an ear of a general adult to measure a dimension (measured value) in an ear canal.
b) Check the insertion/wearing feeling (hit, oppressive feeling, etc.) of a prototype earpiece based on the above dimension.
c) Combine the above a) and b) to determine a size in the ear canal. In addition, each portion has a range due to individual differences.
d) Based on an average value of the size in the ear canal, the dimensions of L1, L2, and L3 are obtained from a role and material of a shape which each portion has.

Here, the dimension of the outer diameter L1 of the tip portion 12b is determined in consideration of the following requirements.
e) The size is such that the eardrum 250 can be oriented by making it thinner toward the second curve C2 in the ear canal 201.
f) At this time, with the intention of softening a contact with the ear canal, a constriction is made between the tip portion 12b and the base portion 12a to make it thinner.
g) If an inner diameter of the recess 20b in which the infrared sensor SN1 is arranged is reduced, the sensitivity (resolution) to infrared rays decreases. Further, assuming that an average inner diameter of the ear canal is set to, for example, 6 mm, the outer diameter L1 of the tip portion 12b needs to be a smaller value than 6 mm.

From the above requirements, the outer diameter L1 of the tip portion 12b is set to, for example, 6 mm or less (preferably about 5.8 mm).

Further, a dimension of the maximum outer diameter L2 of the base portion 12a is determined in consideration of the following requirements.
h) The base portion 12a is not a portion that plays a major role in orienting the probe PB.
i) It is not necessary to seal the ear canal 201 with the base portion 12a.
j) It is sufficient if there is a portion that comes into contact with the ear canal 201 in order to prevent the base portion 12a from coming off.

From the above requirements, the maximum outer diameter L2 of the base portion 12a is set to, for example, about 10 mm ± 2 mm.

Further, a dimension of the total length L3 of the earpiece 12 in the insertion/removal direction into the ear hole is determined in consideration of the following requirements.
k) The distance to reach the second curve C2 in the ear canal 201.
l) The measured value from an entrance of the ear canal 201 to the second curve C2 was in the range of 10 to 14 mm.
m) Inserting the earpiece 12 too much into the ear hole increases a risk of damage to the ear hole.

From the above requirements, a dimension of the total length L3 of the earpiece 12 in the insertion/removal direction into the ear hole is set to, for example, about 10 mm ± 2 mm.

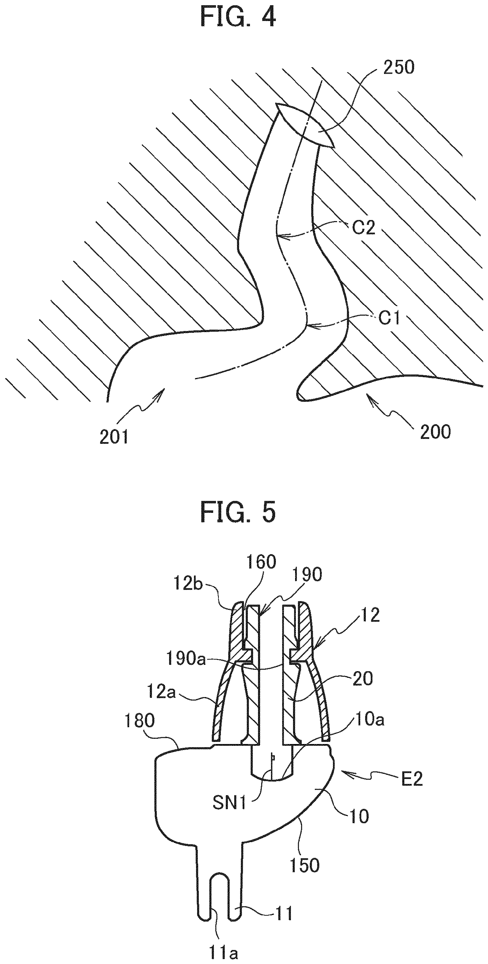

Here, as illustrated in FIG. 4, the ear canal 201 of the ear (right ear) 200 is curved in an S shape so as to prevent an invasion of foreign materials. The curve closer to the entrance is called a first curve C1 and the curve farther from the entrance is called a second curve C2.

The length of the ear canal 201 is such that a distance from the entrance to the eardrum 250 is generally about 25 to 30 mm for adults.

Next, with reference to FIG. 3, a shape and dimensional example of the housing 10 of the ear thermometer E1 according to the first embodiment will be described.

As illustrated in FIG. 3, a curved portion 150 is formed on the right side surface of the housing 10. On the other hand, the left side surface of the housing 10 has a linear shape.

Further, a knob portion (projection portion) 11 for inserting and removing the ear thermometer E1 from an ear hole is formed on the lower end side of the housing 10. At the lower end of the knob portion 11, a groove portion 11a is formed to partially hold a connection cable 13 connected with a measuring device or the like illustrated in FIG. 1(a) or the like.

The shape and dimension of the curved portion 150 and the like of the housing 10 are determined such that the infrared sensor SN1 can stably hold infrared rays in a state where it can efficiently receive the infrared rays from the eardrum 250 by coming into contact with a predetermined position in the ear canal 201.

More specifically, the distance W1 between a lateral line of the curved portion 150 and a parallel line from the left lower end of the base portion 12a of the earpiece 12 is set to, for example, about 10.2 mm.

The distance W2 between the right end of the knob portion 11 and the right end of the curved portion 150 is set to, for example, about 7.8 mm or less based on the distance W1.

The distance W3 between the upper end of the housing 10 and the lower end of the curved portion 150 is set to, for example, about 8.3 mm or less based on the distance W1.

As a result, the ear thermometer E1 is stably held in a state where it can efficiently receive infrared rays from the eardrum 250.

As illustrated in FIG. 2, a gap 160 is provided between the outer peripheral surface of the tip portion of the probe body 20 and the inner peripheral surface of the tip portion 12b of the earpiece 12.

The gap 160 facilitates an inward deformation of the tip portion 12b of the earpiece 12, thereby improving the fit of the earpiece 12 into the ear.

In addition, a space 170 is formed in the base portion 12a, which facilitates an inward deformation, thereby improving the fit of the earpiece 12 into the ear.

### (Modified Example of First Embodiment)

Next, an ear thermometer E2 according to a modified example of the first embodiment will be described with reference to FIG. 5.

Regarding the configuration of the ear thermometer E2 according to the modified example of the first embodiment, since the same components as those of the ear thermometer E1 according to the first embodiment are designated by the same reference numerals, the duplicate description will be omitted.

The difference between the ear thermometer E1 according to the first embodiment and the ear thermometer E2 according to the modified example of the first embodiment lies in that a cylindrical light guide tube 190 in which a reflective material such as gold plating is arranged on an inner wall 190a is used as a probe body.

Further, in the ear thermometer E2 according to the modified example of the first embodiment, the infrared sensor SN1 is arranged in a recess 10a formed on the rear end side of the light guide tube 190.

As a result, the influence of noise on infrared rays from the eardrum 250 can be suppressed, therefore, more accurate body temperature measurement can be performed.

### (Attachment State of Ear Thermometer)

With reference to FIG. 6, an attachment state of the ear thermometer E1 (E2) according to the first embodiment with respect to the ear (right ear) 200 will be described.

As illustrated in FIG. 6, in the ear thermometer E1 (E2) inserted into the ear canal 201 of the ear (right ear) 200, an outer peripheral surface of the tip portion 12b of the earpiece 12 comes into contact with an inner wall of the ear canal near the second curve C2 at the position P1.

As illustrated in FIG. 6, a flat surface portion 180 at the upper left portion of the housing 10 of the ear thermometer E1 (E2) comes into contact with a part of the ear canal 201 near the first curve C1 at the position P2.

As illustrated in FIG. 6, the curved portion 150 at the lower right side of the housing 10 of the ear thermometer E1 (E2) comes into contact with a part of the ear canal 201 near the first curve C1 at the position P3.

In this way, the ear thermometer E1 (E2) comes into contact with a part of the ear canal 201 at three points of positions P1 to P3 in the ear canal 201 of the ear (right ear) 200, therefore, the ear thermometer E1 (E2) is stably held in a state where a center line D1 of the earpiece 12 faces the eardrum 250.

Then, in such a holding state, as illustrated in FIG. 6, the infrared IR emitted from the eardrum 250 is efficiently incident from the tip portion 12b of the earpiece 12. Therefore, the temperature is surely measured by the infrared sensor SN1 illustrated in FIG. 2 or FIG. 5.

As described above, the ear thermometer E1 (E2) according to the first embodiment can be stably held in a state where the infrared IR from the eardrum 250 can be efficiently received.

Here, with reference to FIG. 7, a state in which a general canal type earphone H1 as a comparison target is attached to the ear (right ear) 200 will be briefly described.

When the earphone H1 including a housing 100 and an earpiece 101 is inserted into the ear canal 201 of the ear (right ear) 200, the earpiece 101 is only held near the first curve C1.

Therefore, as illustrated in FIG. 7, a center line D2 of the earpiece 101 of the earphone H1 is in a direction significantly deviated from the eardrum 250. In such a holding state, acoustic effects such as reverberating bass can be obtained by compressing air, but it is not suitable for efficiently capturing infrared IR emitted from the eardrum 250.

Accordingly, when the shape of the housing 100 of the earphone H1 or the general earpiece 101 is applied to the ear thermometer, reliable temperature measurement is not performed unlike the ear thermometer E1 (E2) according to the first embodiment.

### (Second Embodiment)

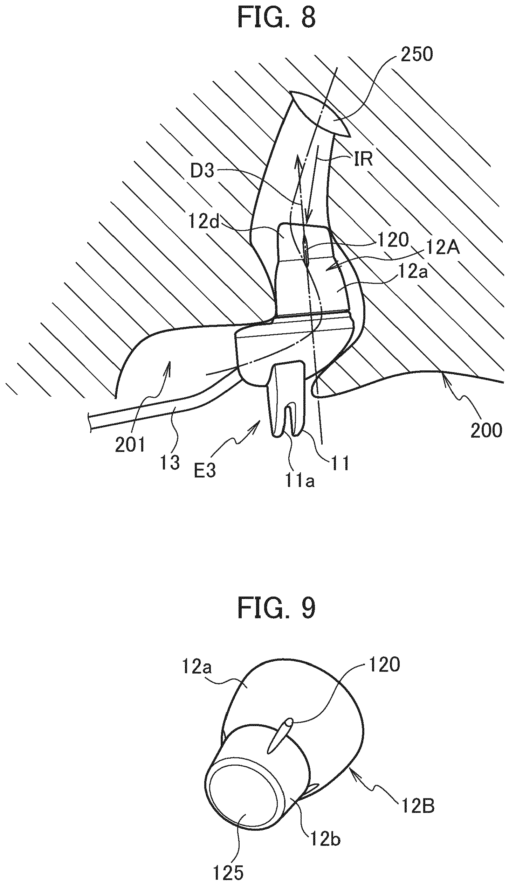

With reference to FIG. 8, an ear thermometer E3 according to a second embodiment will be described.

Regarding the same components as those of the ear thermometer E1 (E2) according to the first embodiment, since the same reference numerals are given, the duplicate explanations will be omitted.

The difference between the ear thermometer E1 (E2) according to the first embodiment and the ear thermometer E3 according to the second embodiment lies in that an earpiece 12A having a different shape is attached instead of the earpiece 12.

More specifically, as illustrated in FIG. 8, a tip portion 12d of the earpiece 12A has a shape in which the right end side is lower than the left end side. With such a shape, the infrared IR from the eardrum 250 can be easily incident from the tip portion 12d of the earpiece 12A, therefore, more accurate temperature measurement can be performed.

### (Third Embodiment)

With reference to FIG. 9, an ear thermometer according to a third embodiment will be described.

The overall configuration of the ear thermometer according to the third embodiment is the same as that of the ear thermometer E1 (E2) according to the first embodiment.

FIG. 9 illustrates an earpiece 12B applied to the ear thermometer according to the third embodiment.

In the earpiece 12B applied to the ear thermometer according to the third embodiment, since the same components as those of the earpiece 12 applied to the ear thermometer E1 (E2) according to the first embodiment are designated by the same reference numerals, the duplicate description will be omitted.

An end face of the tip portion 12b of the earpiece 12B is closed by a protective film 125.

The protective film 125 is integrally formed with the tip portion 12b by silicone rubber or the like.

A thickness of the protective film 125 is set to, for example, 0.2 mm or less.

By providing such a protective film 125, it is possible to suppress the influence of thermal noise due to the fluctuation of air in the earpiece 12B, thereby performing more accurate body temperature measurement.

### (Fourth Embodiment)

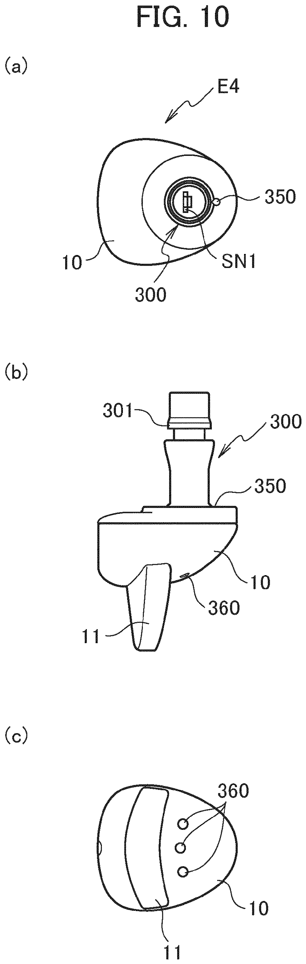

With reference to FIGS. 10 to 12, an ear thermometer E4 (E5) according to a fourth embodiment will be described.

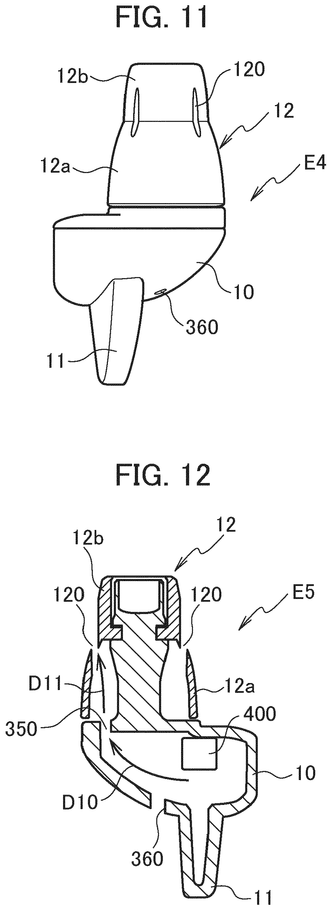

Here, FIG. 10(a) is a top view illustrating a configuration example of the ear thermometer E4 according to the fourth embodiment, FIG. 10(b) is a right side view illustrating a configuration example of the ear thermometer E4 according to the fourth embodiment, and FIG. 10(c) is a bottom view illustrating a configuration example of the ear thermometer E4 according to the fourth embodiment. FIG. 11 is a right side view illustrating a state in which the earpiece 12 is attached to the ear thermometer E4 according to the fourth embodiment. FIG. 12 is a cross-sectional view illustrating a configuration example of the ear thermometer E5 according to a modified example of the fourth embodiment.

In the ear thermometer E4 (E5) according to the fourth embodiment, since the same components as those of the ear thermometer E1 (E2) according to the first embodiment are designated by the same reference numerals, the duplicated explanations will be omitted.

The difference between the ear thermometer E1 (E2) according to the first embodiment and the ear thermometer E4 (E5) according to the fourth embodiment lies in that the housing 10 includes external sound intake holes 360 that takes in an external sound on the probe body 300.

As illustrated in FIG. 10(c), in the fourth embodiment, three external sound intake holes 360 are bored on a bottom surface of the housing 10.

This makes it possible to transmit an external instruction or the like to a temperature measurement target person by sound.

Further, the ear thermometer E4 (E5) according to the fourth embodiment includes a sound output hole 350 on the upper side of the housing 10. The sound output hole 350 emits a sound taken in from the external sound intake holes 360 to the earpiece 12.

Further, as illustrated in FIG. 11 and the like, the earpiece 12 has sound emitting portions 120 that emit a sound emitted from the sound output hole 350 into the ear hole.

This makes it possible to more reliably transmit a sound such as an external instruction to a temperature measurement target person.

Further, as illustrated in FIG. 12, in an ear thermometer E5 according to a modified example of the fourth embodiment, a sound output unit (for example, a small speaker) 400 is provided in the housing 10. The sound output unit 400 outputs a sound by being connected to an external device (not illustrated) such as a synthetic sound output device or a microphone.

At this time, in the housing 10, the sound and the like are output in the direction of the arrows D10 and D11.

As a result, an instruction or an alarm can be transmitted more clearly to a temperature measurement target person by outputting the instruction or the alarm from the sound output unit 400 by sound via the external device.

Although the ear thermometer according to the present invention has been described based on the illustrated embodiments, the present invention is not limited to these, and the configuration of each portion may be replaced with any configuration having the same function.

For example, in each embodiment, an example in which the ear thermometer is attached to a right ear of a temperature measurement target is illustrated, but the shape may be changed so that the thermometer can be attached to a left ear of a temperature measurement target.

## Claims

1. An ear thermometer, comprising:
a probe comprising an infrared sensor configured to measure a temperature of an eardrum of a temperature measurement target person in a non-contact manner and configured to be attached to an ear hole of the temperature measurement target person, wherein
the probe comprises:
a probe body configured to be inserted into the ear hole of the temperature measurement target person;
a housing supporting the probe body; and
an in-ear type earpiece attached to the probe body and configured to come into contact inside the ear hole of the temperature measurement target person,
the earpiece comprises:
a base portion comprising an engaging portion configured to engage with the probe body and partially exhibiting a hollow conical shape; and
a substantially cylindrical tip portion provided at one end of the base portion and extending in a direction away from the housing,
the base portion and the tip portion are integrally formed of a flexible material, and
an outer diameter of the tip portion is set to be smaller than a maximum outer diameter of the base portion.

2. The ear thermometer of claim 1, wherein
a total length of the earpiece in an insertion/removal direction into the ear hole is set to have a length that reaches a second curve located on an inner side of an S-shaped curve of an ear canal.

3. The ear thermometer of claim 1 or 2, wherein
the infrared sensor is arranged in a recess formed on a tip side of the probe body.

4. The ear thermometer of claim 1 or 2, wherein
the probe body includes a cylindrical light guide tube in which a reflective material is arranged on an inner wall, and
the infrared sensor is arranged on a rear end side of the light guide tube.

5. The ear thermometer of any one of claims 1 to 4, wherein
the housing includes an external sound intake hole for taking in an external sound on the probe body.

6. The ear thermometer of claim 5, wherein
the housing includes a sound output hole for emitting a sound taken in from the external sound intake hole to the earpiece, and
the earpiece is provided with a sound emitting portion for emitting a sound emitted from the sound output hole into the ear hole.

7. The ear thermometer of claim 5 or 6, further comprising
a sound output unit provided in the housing and configured to output a sound by being connected to an external device.

8. The ear thermometer of any one of claims 1 to 7, wherein
the tip portion of the earpiece is closed by a protective film.
